# EUROPEAN PATENT SPECIFICATION

(11) **EP 2 647 356 B1**
(45) Date of publication and mention of the grant of the patent: **23.09.2020**
(21) Application number: 13161593.2
(22) Date of filing: 28.03.2013
(51) Int. Cl.: A61F 2/68, F16K 17/30, A61F 2/74, A61F 2/50

(54) **An multi-stage air pressure valve disposed in the airway of a prosthesis air cylinder**
Mehrstufiges Luftdruckventil das im Atemweg eines Prothesenluftzylinders angeordnet ist
Soupape de pression d'air multi-niveaux disposée dans les voies respiratoires d'un cylindre d'air de prothèse

(30) Priority: 05.04.2012 TW 101206148
(43) Date of publication of application: 09.10.2013
(73) Proprietor: PRO LIMB International Corp., New Taipei City 238 (TW)
(72) Inventor: Shen, Hsin Fa, Taipei County 220 (TW)
(74) Representative: Cabinet Chaillot

(56) References cited:
- WO-A1-02/084423
- DE-C- 332 451
- GB-A- 2 037 406
- GB-A- 2 328 160
- US-A- 2 842 159
- US-A- 5 085 246
- US-A1- 2006 000 510

## Description

### Technical Field

The present creation relates to a cushioning air cylinder, especially to an adjustment-free multi-stage prosthesis air cylinder which has a multi-stage air pressure valve and can be used in prosthesis joint.

### Background Art

The air cylinder is a structure which controls air pressure by using the piston's stretch-out and draw-back. It has characteristics of accepting accumulation of pressures and automatically returning after removing of external forces due to the compressibility of air pressure.

The air cylinder is also applied to prosthesis joint and used as medical device providing cushioning function as an alternative of joint. In the common design of the prosthesis joint, the cushioning air cylinder provides cushioning function for certain compression strength. For example, a cushioning air cylinder that corresponds to a slow walking speed is used for the user who slowly walks; while the user wants to conduct a brisk walk or go jogging, it must be replaced by another cushioning air cylinder which adapts to that brisk walk speed. In other words, the user must prepare two or more kinds of cushioning air cylinders in order to be adapted to different walking speeds.

Another relatively new type of cushioning air cylinder has a stepped device to set the level of the cushioning force. The users themselves can adjust the air cylinder setting according to the walking speeds so that the cushioning air cylinder can generate corresponding cushioning capacity.

However, although this type of air cylinder can be individually used to respond to the changes of the environment, the user still needs to actively adjust the device whenever the walking speed is changed, whatever modification is made to the adjustment mode. And, under any condition where a wrong cushioning mode is set, it may not only cause the damage of the cushioning air cylinder, but also more likely to cause the user uncomfortable or even injured for the lack of cushioning capacity.

A conventional pressure valve, such as COMBINATION SURGE SUPPRESSOR AND SAFETY SHUT-OFF VALVE disclosed in PCT Patent Publication No. WO 02/084423 A1, comprises a valve body, and a poppet and a spring disposed in the valve body. The valve body has an inlet port, an outlet port, and a valve seat formed around the outlet port. The poppet has a base with multiple flow apertures, a neck extending from the base, an enlarged head formed on a distal end of the neck, and a bypass passageway formed through the base, the neck, and the head. The spring abuts against the valve seat of the valve body and the base of the poppet, so as to urge the poppet to slide away from the valve seat. Fluid flows in the inlet port of the valve body, passes through the flow apertures and the bypass passageway of the poppet, and then flows out of the valve body via the outlet port of the valve body. Moreover, the fluid also urges the poppet to slide toward the valve seat of the valve body. As the fluid exceeds a predetermined pressure, the head of the poppet is urged to abut against the valve seat, the outlet port is blocked, and the fluid can only pass through the bypass passageway to flow out of the valve body. Accordingly, flow rate of the fluid can be reduced.

Another conventional pressure valve, such as VALVE disclosed in GB Patent Publication No. 2037406, includes a valve seat, a valve member, and a resilient member mounted around the valve member. The valve seat has a bore portion. The valve member is mounted in the bore portion of the valve seat and has a cylindrical portion, a conical portion, a blind bore, an axial passage leading from the blind bore to an apex of the conical portion, and multiple side passages extending from the blind bore to a conical surface of the conical portion. The resilient member abuts against the valve seat and tends to urge the valve member to slide away from the housing. Gas flows into the blind bore of the valve member, passes through the axial passage and the side passages, and then flows out of the valve seat via the bore portion of the valve seat. The Gas also urges the valve member to slide toward the valve seat. As the gas exceeds a predetermined pressure, the conical portion of the valve member is urged to abut against the valve seat, the side passages are blocked, and the gas can only pass through the axial passage to flow out of the valve seat. Accordingly, flow rate of the gas can be reduced.

Still another conventional pressure valve, such as plural-rate surge-suppressing valve disclosed in US Patent No. 5,085,246, has a valve body, and a valve member and a helical spring mounted in the valve body. The valve body has an inlet chamber and an outlet chamber. The valve member is disposed between the inlet chamber and the outlet chamber and has an axial passage and multiple side passages. The helical spring abuts against the valve body and the valve member, so as to urge the valve member to slide away from the outlet chamber. Fluid flows in the inlet chamber of the valve body, passes through the axial passage and the side passages of the valve member, and the flows out of the valve body via the outlet chamber of the valve body. The fluid also urges the valve member to slide toward the outlet chamber. As the fluid exceeds a predetermined pressue, the valve member is urged to abut against the valve body, the side passages are blocked, and the fluid can only pass through the axial passage to flow out of the valve body. Accordingly, flow rate of the fluid can be reduced.

However, although the above-mentioned conventional pressure valves can reduce the flow rates of the fluid or the gas passing therethrough, the above-mentioned conventional pressure valves cannot cushion movements of the poppet or the valve members therein.

### Summary of the Invention

The present creation intends to provide an adjustment-free multi-stage prosthesis air cylinder having a multi-stage air pressure valve, which can be used in prosthesis joint to be automatically adapted to different impact strength under different motion speeds of the joint, so as to improve the environmental adaptability as well as the life span of the prosthesis joint.

The present creation provides a multi-stage air pressure valve 1 which is disposed in the air way comprising a first component, a second component, two sealing rings and an elastic piece with the inner of the first component including a main channel and a first bypass channel. The main channel penetrates through the first component, forms an air inlet on the top surface of the first component and a first air outlet on the bottom surface of the first component. The first bypass channel communicates with the main channel and forms a second air outlet on the side surface of the first component. The second component is sleeved on the first component. A second bypass channel communicating with the first bypass channel is formed between the first component and the second component. The two sealing rings are sleeved on the side surface of the first component to optionally close the second bypass channel, and the second air outlet is located between the two sealing rings. The elastic piece connects the first component with the second component.

The present creation further provides an adjustment-free multi-stage prosthesis air cylinder comprising an air cylinder body, a piston, a first check valve and a multi-stage air pressure valve. An air chamber is formed inside the air cylinder body, and the cylinder body forms a lower air way. One end of the piston is slidably disposed in the air chamber, and the other end of the piston extends out of the air cylinder body. The piston divides the air chamber into an upper air chamber and a lower air chamber. An upper air way is formed inside the piston, having one end connecting to the outside and the other end connecting to the upper air chamber. The lower air chamber communicates with the lower air way, and the piston makes a reciprocating movement in the air chamber. The first check valve is disposed in the piston to optionally connect the upper air chamber with the lower air chamber. The multi-stage air pressure valve that is disposed inside the lower air way comprises a first component, a second component, two sealing rings and an elastic piece. The inner of the first component includes a main channel and a first bypass channel, the main channel penetrates through the first component, forming an air inlet on the top surface of the first component and a first air outlet on the bottom surface of the first component. The first bypass channel communicates with the main channel and forms a second air outlet on the side surface of the first component. The second component is sleeved on the first component, and a second bypass channel communicating with the first bypass channel is formed between the first component and the second component. The two sealing rings are sleeved on the side surface of the first component to optionally close the second bypass channel, and the second air outlet is formed between the two sealing rings; the elastic piece connects the first component with the second component.

The present creation is beneficial in that the adjustment-free multi-stage prosthesis air cylinder of the present creation is suitable for prosthesis joint, mainly suitable for knee prosthesis for legs; when the users are slowly walking (usually with a walking speed less than 2∼4 km/h), the gas flow rate is relatively larger, so as to quickly discharge the inside pressure, which enables a longer cushioning stroke for the piston so that the users can feel more comfortable. When the users are walking in a high speed (usually with a walking speed around 4∼8 km/h), the gas flow rate of the multi-stage air pressure valve 1 is automatically reduced so that the inner thereof has an instantaneous high pressure, which enables a shorter cushioning stroke for the piston to provide the users with a good operability.

The adjustment-free multi-stage prosthesis air cylinder of the present creation is simple to install, so that the users do not need to adjust the setting of the air pressure valve but only need to assemble the adjustment-free multi-stage prosthesis air cylinder with the prosthesis joint; moreover, since the adjustment-free multi-stage prosthesis air cylinder has a simple structure, lower cost, and longer span lift, it overcomes the defects of the traditional installation that is complex, error-prone and requires frequent maintenance.

The features and the technical contents of the present creation will be further appreciated from the following detailed description and figures which are illustrated for reference and explanation only but not to limit the extent of the scope of the present creation.

### Brief Description of the Figures

Figure 1A is a cross-section schematic diagram of the first component before compression of the first embodiment of the present creation.
Figure 1B is a cross-section schematic diagram of the first component after compression of the first embodiment of the present creation.
Figure 2 is a cross-section schematic diagram of the first component before compression of the second embodiment of the present creation.
Figure 2A is a cross-section schematic diagram of the first component during compression of the second embodiment of the present creation.
Figure 2B is a cross-section schematic diagram of the first component after compression of the second embodiment of the present creation.
Figure 3 is a cross-section schematic diagram of the third embodiment of the present creation.
Figure 3A is a schematic diagram showing the airflow when the piston is moving upwards of the third embodiment of the present creation.
Figure 3B is a schematic diagram showing the airflow when the piston is moving downwards of the third embodiment of the present creation.
Figure 4 is a cross-section schematic diagram of the third embodiment of the present creation.

**List of Reference Numerals**

| | |
|---|---|
| the multi-stage air pressure valve | 1, 1' |
| the first component | 11 |
| the top part | 111 |
| the protrusion part | 112, 112' |
| the main channel | 113 |
| the air inlet | 1131 |
| the first air outlet | 1132 |
| the first bypass channel | 114 |
| the second air outlet | 1141 |
| the second component | 12 |
| the bottom part | 121 |
| the extension part | 122 |
| the sealing ring | 13 |
| the elastic piece | 14 |
| the second bypass channel | 15 |
| the air cylinder body | 2 |
| the air chamber | 21 |
| the upper air chamber | 211 |
| the lower air chamber | 212 |
| the lower air way | 22 |
| the piston | 3 |
| the upper air way | 31 |
| the first check valve | 4 |
| the second check valve | 5 |

The adjustment-free multi-stage prosthesis air cylinder of the present creation uses the multi-stage air pressure valve which is disposed in the air passage to generate cushioning effect and supporting force corresponding to different pressures.

### Embodiment 1

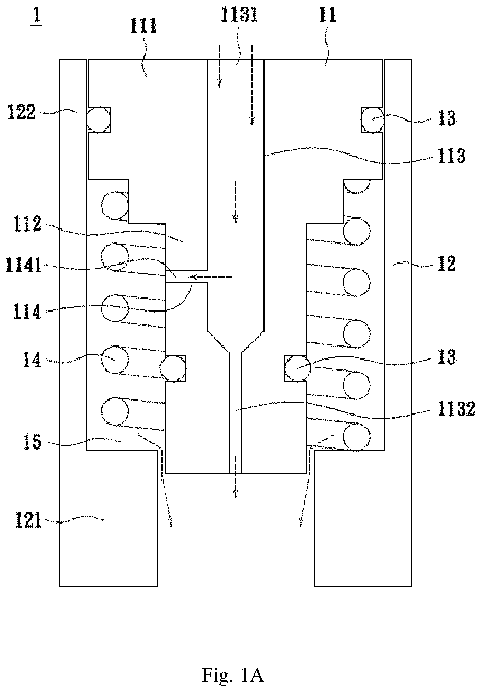

Referring to figure 1A, the present embodiment provides a multi-stage air pressure valve 1 which includes a first component 11, a second component 12, an elastic piece 14 and two sealing rings 13. The first component 11 includes two main parts, which are a top part 111 and a protrusion part 112 extending from the top part 111, respectively. A main channel 113 is formed inside the first component 11, penetrating there-through; the main channel 113 forms an air inlet 1131 on the top surface of the first component 11 and a first air outlet 1132 on the bottom surface of the first component 11. The inner of the first component 11 further forms a first bypass channel 114, which has one end connecting to the main channel 113, and the other end penetrating through the first component 11 and forming a second air outlet 1141 on the side surface of the first component 11.

The second component 12 is sleeved on the first component 11, and a second bypass channel 15 is formed between the first component 11 and the second component 12. In details, the second component 12 includes a bottom part 121 and an extension part 122, the extension part 122 radially extends along the direction of the first component 11 and surrounds the top part 111 of the first component 11. The first component 11 slides in the inner of the second component 12, and the protrusion part 112 of the first component 11 optionally slides into the bottom part 121 of the second component 12. Two sealing rings 13 are disposed on the first component 11, in which one is sleeved on the protrusion part 112 and the other one is sleeved on the top part 111; a second air outlet 1141 is located between the two sealing rings 13.

The second bypass channel 15 that is formed between the first component 11 and the second component 12 is optionally sealed by the sealing ring 13. In details, one sealing ring 13 seals the gap between the top part 111 and the extension part 122, and the other sealing ring 13 seals the gap between the protrusion part 112 and the bottom part 121.

An elastic piece 14 is disposed in the sliding direction of the first component 11 and of the second component 12; the elastic piece 14 may be a spring which surrounds the protrusion part 112 of the first component 11, one end of the elastic piece is connecting to the top part 111 of the first component 11, and the other end is connecting to the bottom part 121 of the second component 12.

Referring to figure 1A for the operational pattern of the multi-stage air pressure valve 1 of the present creation, when the air enters the air inlet 1131, with the first component 11 not subjected to compressing force, it passes through the main channel 113 and exits from the first outlet 1132. The second bypass channel 15 is in an on-state due to the tap between the protrusion 112 and the bottom 121, so that the air also exits the multi-stage air pressure valve 1 via the second bypass channel 15. Since the main channel 113, the first bypass channel 114 and the second bypass channel 15 are all in an on-state, the multi-stage air pressure valve 1 then has a relatively higher gas flow rate.

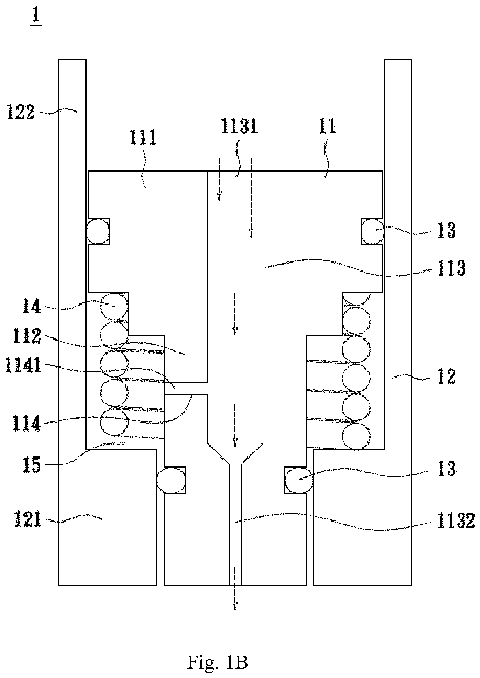

Referring to figure 1B, the first component 11 is subjected to compressing force and slides deeply into the second component 12; under such state, the air enters from the air inlet 1131, passes the main channel 113 and exits from the first air outlet 1132; However, the air in the second side channel 15 can not be discharged because the gap between the protrusion part 112 and the bottom part 121 is sealed. In other words, when the multi-stage air pressure valve 1 is subjected to a certain compressing force which causes the second bypass channel 15 being sealed by the sealing ring 13, the air can only exits the multi-stage air pressure valve 1 via the first air outlet 1132 of the main channel 113, so that the gas flow rate of the multi-stage air pressure valve 1 under compressing force is lower than where there is no compressing force.

It should be supplemented that the sealing ring 13 is disposed between the top part 111 and the extension part 122 for preventing the air of the second bypass channel 15 from couter-flowing to the outside via that gap.

### Embodiment 2

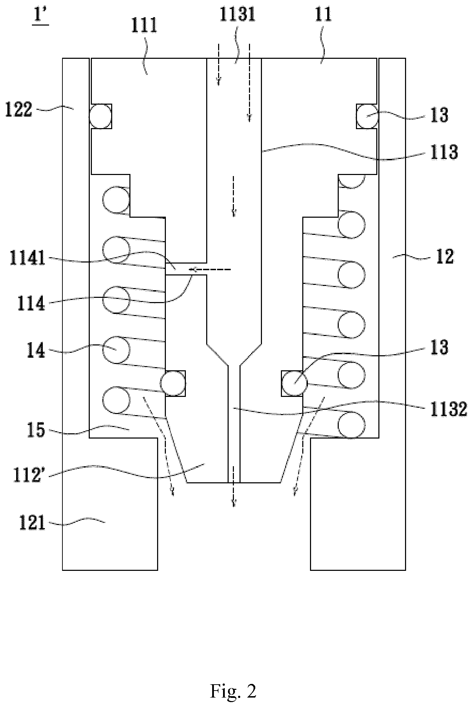

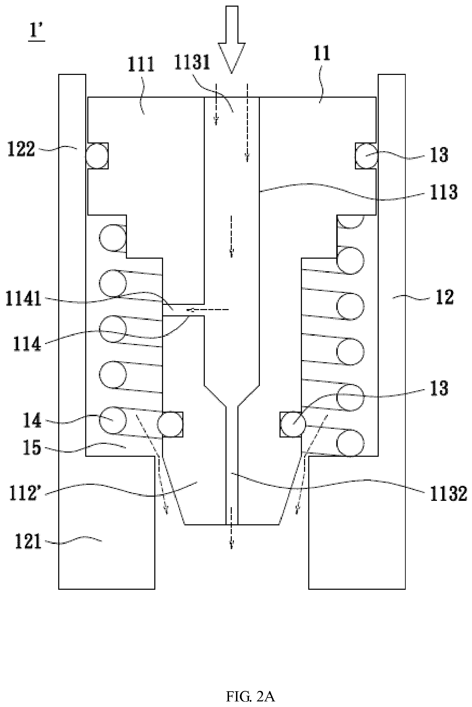

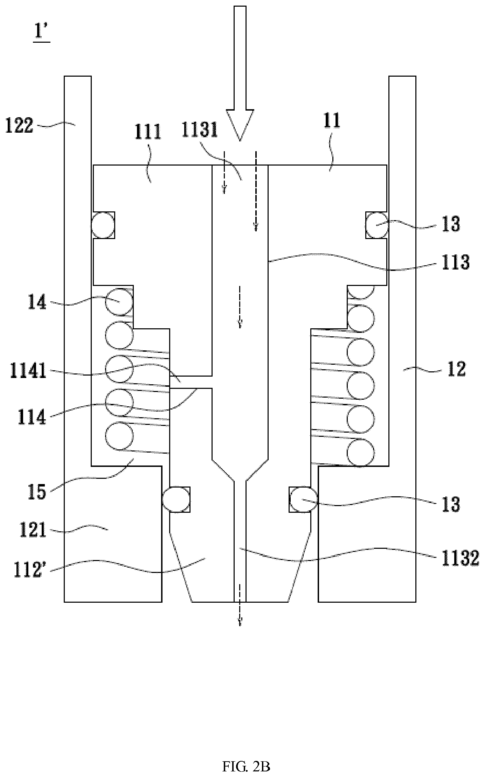

An embodiment of another multi-stage air pressure valve 1 of the present creation is as shown in figure 2. The second embodiment is different from the first embodiment in that the protrusion part 112' of the first component 11 forms into a tapered shape between the second air outlet 1141 and the first air outlet 1132; in other words, the first component 11 and second component 12 form different gaps there-between depending on the different relative positions thereof. The sealing ring 13 is disposed on the starting point of the tapered shape, to optionally seal the gap between the protrusion part 112' and the bottom part 121.

The operational pattern of the present embodiment is shown in figure 2. The first component 11 is subjected to the compressing force and then starts to slide towards the bottom part 121 of the second component 12. At this time, the second bypass channel 15 has a relatively higher gas flow rate due to the greater gap between the protrusion part 112' and the bottom part 121. As shown in figure 2A, the gap between the protrusion part 112' and the bottom part 121 is gradually narrowed as the protrusion part 112' of the first component 11 is gradually moved into the bottom part 121 of the second component 12. In other words, the gas flow rate of the second bypass channel 15 is gradually reduced. As shown in figure 2B, when the sealing ring 13 seals the gap between the protrusion part 112' and the bottom part 121, the gas flow rate of the second bypass channel 15 is dropped to zero.

As mentioned above, the gas flow rate of the multi-stage air pressure valve 1 of the present embodiment is provided by the main channel 113, the first bypass channel 114, and the second bypass channel 15. The gas flow rate of the second bypass channel 15 is gradually reduced as the first component 11 is subjected to the compressing force and gradually merged into the second component 12. In other words, the multi-stage air pressure valve 1 of the present embodiment provides different gas flow rates according to different compressing forces to obtain multi-stage adjustment of the air pressure valve.

### Embodiment 3

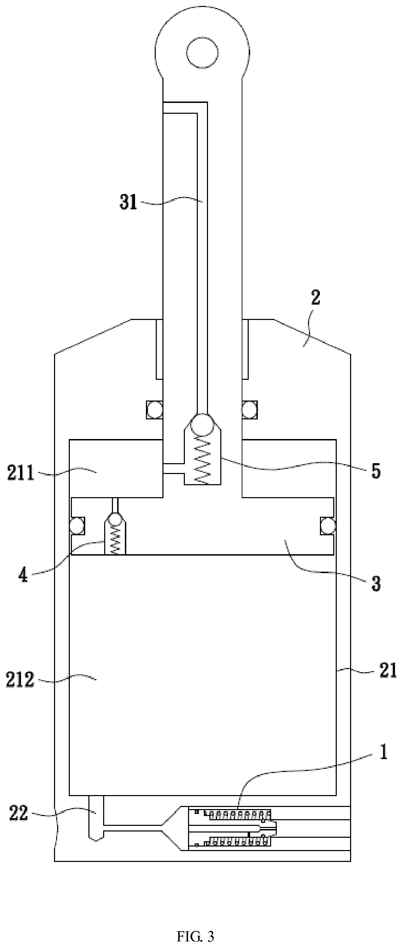

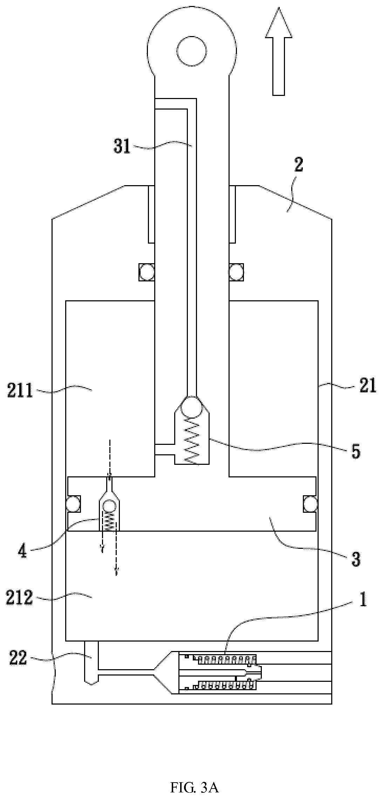

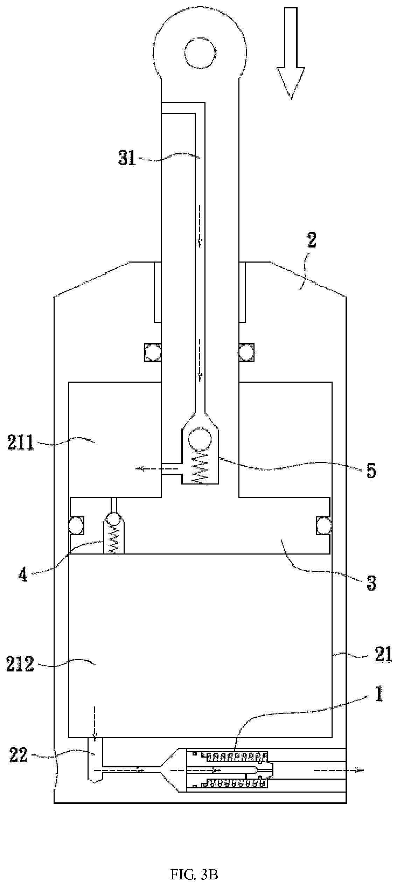

As shown in figure 3, the present embodiment provides an adjustment-free multi-stage prosthesis air cylinder having a multi-stage air pressure valve 1 which can be used in prosthesis joint, the adjustment-free multi-stage prosthesis air cylinder comprises an air cylinder body 2, a piston 3, a first check valve 4 and a multi-stage air pressure valve 1.

An air chamber 21 is formed inside the air cylinder body 2, and the piston 3 is disposed inside the air chamber 21.

The piston 3 externally extends out of the cylinder body 2. One end of the piston 3 is slidably disposed inside the air chamber 21, and the other end of the piston 3 extends to the outside of the air cylinder body 2. The piston 3 divides the air chamber 21 into an upper air chamber 211 and a lower air chamber 212, and the piston 3 optionally slides in the air chamber 21 to make a reciprocating movement, so that volumes of the upper air chamber 211 and the lower air chamber 212 are variable.

The first check valve 4 is disposed in the piston 3, having one end connecting to the upper air chamber 211, and the other end connecting to the lower air chamber 212. The air in the upper air chamber 211 can enter the lower air chamber 212 through the first check valve 4, but the air in the lower air chamber 212 can not counter-flow into the upper air chamber 211 due to the stop function of the first check valve 4.

An upper air way 31 is formed inside the piston 3, the two ends of the upper air way 31 are connected to the outside and to the upper air chamber 211, respectively, and a second check valve 5 is disposed inside the upper air way 31. A lower air way 22 is formed inside the air cylinder body 2, and the two ends of the lower air way 22 are connected to the outside and to the lower air chamber 212, respectively.

As shown in figure 3A, when the piston 3 moves upwards, the volume of the upper air chamber 211 is gradually reduced and the volume of the lower air chamber 212 is gradually increased. At this moment, the air pressure of the upper air chamber 211 rises, the first check valve 4 is subjected to a pressure difference and opened, while the second check valve 5 is closed to allow the air from the upper air chamber 211 filling the lower air chamber 212. As shown in figure 3B and figure 1A, when the piston 3 slowly moves downwards, the volume of the upper air chamber 211 is increased and the volume of the lower air chamber 212 is reduced. At this moment, the air pressure of the upper air chamber 211 drops, the second check valve 5 is opened to allow the external air filling the upper air chamber 211, while the first check valve 4 is closed so that the air in the lower air chamber 212 is compressed by the piston 3, flows into the lower air way 22 and is discharged from the air cylinder body 2 through the main channel 113, the first bypass channel 114 and the second bypass channel 15 of the multi-stage air pressure valve 1. As shown in figure 3B and figure 1B, when the piston 3 rapidly moves downwards, an instantaneous high pressure pushes the multi-stage air pressure valve 1 so that the first component 11 is subjected to a greater downward force to compress the elastic piece 14, which makes the protrusion 112 being moved into the bottom part 121 of the second component 12. Since the gap between the protrusion part 112 and the bottom part 121 is sealed by the O-shaped ring, the second bypass channel 15 of the multi-stage air pressure valve 1 is blocked, so that the air that is compressed downwards can only be discharged from the first air outlet 1132 of the main channel 113.

In conclusion, when the piston 3 slowly reciprocates, the air in the lower air chamber 212 is discharged through the main channel 113, the first bypass channel 114 and the second bypass channel 15 of the multi-stage air pressure valve 1 (figure 1A) with a longer cushioning stroke so that the user can feel more comfortable. When the piston 3 rapidly reciprocates, the air in the lower air chamber 212 can only be discharged through the main channel 113 of the multi-stage air pressure valve 1 (figure 1B) with a shorter cushioning stroke so that the user is highly sensitive to the external environment (such as road surface).

### Embodiment 4

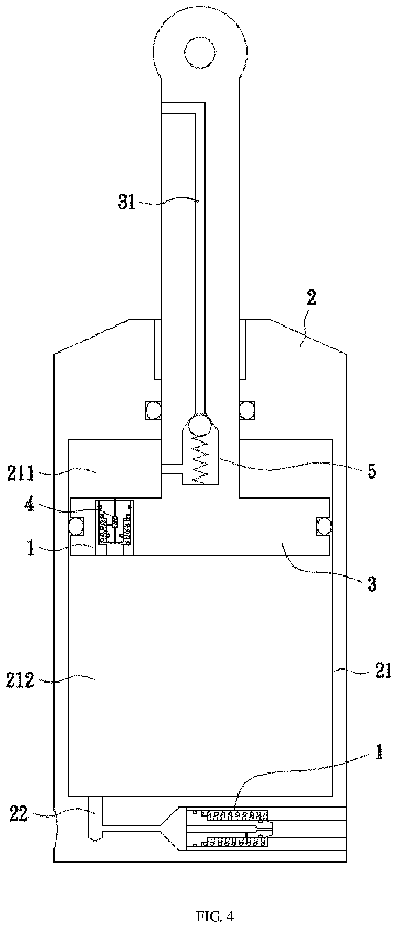

As shown in figure 4, the fourth embodiment is different from the third embodiment in that a multi-stage air pressure valve 1 is disposed inside the piston 3, and the first check valve 4 is disposed inside the multi-stage air pressure valve 1.

The airflow can be preliminarily adjusted when passing through the piston 3, and further adjusted for its gas flow rate via the multi-stage pressure valve 1 in the lower air way 22, so that the adjustment-free multi-stage prosthesis air cylinder having multiple stages is realized by the mutual-combination of the multi-stage air pressure valves 1 disposed in the piston 3 and in the lower air way 22.

### Potential Effects of the Embodiments

The adjustment-free multi-stage prosthesis air cylinder of the present creation is suitable for prosthesis joint, mainly suitable for knee prosthesis for legs. When the users are slowly walking (usually with a walking speed less than 2∼4 km/h), the gas flow rate is relatively larger, so as to quickly discharge the inside pressure, which enables a longer cushioning stroke for the piston so that the users can feel more comfortable. When the users are walking in a high speed (usually with a walking speed around 4∼8 km/h), the gas flow rate of the multi-stage air pressure valve 1 is automatically reduced so that the inner thereof has an instantaneous high pressure, which enables a shorter cushioning stroke for the piston to provide the prosthesis with a good operability.

The adjustment-free multi-stage prosthesis air cylinder of the present creation is simple to install, so that the users do not need to adjust the setting of the pressure valve, but only need to assemble the adjustment-free multi-stage prosthesis air cylinder with the prosthesis joint, without requirements to check and adjust the pressure valve. Moreover, since the adjustment-free multi-stage prosthesis air cylinder has a simple structure, lower cost, and longer span lift, it overcomes the defects of the traditional installation that is complex, error-prone and requires frequent maintenance.

It should be stated that the above description only illustrates the preferred embodiments of the present creation and is not intended to limit the extent of scope thereof. Therefore all the equivalent changes by following the concepts of the specification and the drawings of the present creation should be fallen within the claimed extent of scope thereof.

## Claims

1. A multi-stage air pressure valve (1, 1') disposed in the air way of a prosthesis air cylinder, the multi-stage air pressure valve (1, 1') comprising:
a first component (11) comprising a top part (111) and a protrusion part (112, 112'), wherein the protrusion part (112, 112') axially extends from the top part (111), an inner of the first component (11) comprises a main channel (113) and a first bypass channel (114), wherein the main channel (113) penetrates through the first component (11) from the top part (111) to the protrusion part (112, 112'), forms an air inlet (1131) on a top surface of the top part (11) of the first component (11) and forms a first air outlet (1132) on a bottom surface of the protrusion part (112, 112') of the first component (11), and wherein the first bypass channel (114) communicates with the main channel (113) and forms a second air outlet (1141) on a side surface of the first component (11);
a second component (12) comprising a bottom part (121), an extension part (122), and an outlet channel, wherein the protrusion part (112, 112') of the first component (11) extends towards the bottom part (121), the extension part (122) axially extends from the bottom part (121) towards the top part (111) of the first component (11), the outlet channel penetrating through the extension part (122), the second component (12) is sleeved on the first component (11), and a second bypass channel (15) communicating with the first bypass channel (114) is formed between the protrusion part (112, 112') of the first component (11) and the second component (12);
a first sealing ring (13) disposed around a side surface of the top part (111) of the first component (11), abutting against the extension part (122) of the second component (12), and sealing a first gap defined between the top part (111) and the extension part (122) and
an elastic piece (14) surrounding the protrusion part (112, 112') of the first component (11) and having two ends, and the ends of the elastic piece (14) are respectively connected to the top part (111) of the first component (11) and the bottom part (121) of the second component (12); the multi-stage air pressure valve (1, 1') **characterized in that**:
a widest radial width of the protrusion part (112, 112') of the first component (11) is smaller than a radial width of the outlet channel of the second component (12) such that the protrusion part (112, 112') is capable of sliding back and forth in the outlet channel;
the multi-stage air pressure valve (1, 1') futher comprises a second sealing ring (13), the second sealing ring (13) is disposed around a side surface of the protrusion part (112, 112') of the first component (11); and
the second air outlet (1141) is located between the first and the second sealing rings (13); wherein
before the first component (11) is compressed, the second sealing ring (13) is positioned out of the outlet channel of the second component (12) and the second bypass channel (15) is open; and
when the first component (11) is compressed, the protrusion part (112, 112') of the first component (11) slides toward the outlet channel of the second component (12) and the second bypass channel (15) is closed as the second sealing ring (13) slides in the outlet channel and abuts against the bottom part (121) of the second component (13) to seal a second gap defined between the protrusion part (112, 112') and the bottom part (121).

2. The multi-stage air pressure valve (1') of claim 1, wherein the first component (11) forms into a tapered shape between the second air outlet (1141) and the first air outlet (1132) so as to form variable gaps between the first component (11) and the second component (12).

3. An adjustment-free multi-stage prosthesis air cylinder applicable for a prosthesis joint, comprising:
a multi-stage air pressure valve (1, 1') as claimed in claim 1;
an air cylinder body (2) forming a lower air way (22), wherein an air chamber (21) is formed inside the air cylinder body (2);
a piston (3), wherein one end of the piston (3) is slidably disposed in the air chamber (21), and the other end of the piston (3) extends out of the air cylinder body (2), wherein the piston (3) divides the air chamber (21) into an upper air chamber (211) and a lower air chamber (212), wherein an upper air way (31) is formed inside the piston (3), one end of the upper air way (31) is connected to the outside and the other end of the upper air way (31) is connected to the upper air chamber (211), wherein the lower air chamber (212) communicates with the lower air way (22) and wherein the piston (3) being adapted to make a reciprocating movement in the air chamber (21);
a first check valve (4), wherein the first check valve (4) is disposed in the piston (3) to optionally connect the upper air chamber (211) with the lower air chamber (212); and wherein the multi-stage air pressure valve (1, 1') is disposed in the lower air way (22).

4. The adjustment-free multi-stage prosthesis air cylinder of claim 3, further comprising a second check valve (5), wherein the second check valve (5) is disposed in the upper air way (31) so that the air can only flow from the outside of the air cylinder body (2) to the upper air chamber (211) in one-way.

5. The adjustment-free multi-stage prosthesis air cylinder of claim 3, wherein the multi-stage air pressure valve (1, 1') is provided with at least a second sealing ring to close the gap between the multi-stage air pressure valve and the lower air way (22).

## Patentansprüche

1. Mehrstufiges Luftdruckventil (1, 1'), das im Luftweg eines Prothesen-Luftzylinders angeordnet ist, wobei das mehrstufige Luftdruckventil (1, 1') umfasst:
eine erste Komponente (11), die einen oberen Teil (111) und einen Vorsprungsteil (112, 112') umfasst, wobei der Vorsprungsteil (112, 112') sich axial vom oberen Teil (111) erstreckt, ein Inneres der ersten Komponente (11) einen Hauptkanal (113) und einen ersten Umgehungskanal (114) umfasst, wobei der Hauptkanal (113) vom oberen Teil (111) durch die erste Komponente (11) zum Vorsprungsteil (112, 112') durchtritt, einen Lufteinlass (1131) auf einer oberen Oberfläche des oberen Teils (11) der ersten Komponente (11) bildet und einen ersten Luftauslass (1132) auf einer unteren Oberfläche des Vorsprungsteils (112, 112') der ersten Komponente (11) bildet, und wobei der erste Umgehungskanal (114) mit dem Hauptkanal (113) in Verbindung steht und einen zweiten Luftauslass (1141) auf einer Seitenfläche der ersten Komponente (11) bildet;
eine zweite Komponente (12), die einen unteren Teil (121), einen Verlängerungsteil (122) und einen Auslasskanal umfasst, wobei der Vorsprungsteil (112, 112') der ersten Komponente (11) sich zum unteren Teil (121) erstreckt, der Verlängerungsteil (122) sich vom unteren Teil (121) axial zum oberen Teil (111) der ersten Komponente (11) erstreckt, der Auslasskanal durch den Verlängerungsteil (122) durchtritt, die zweite Komponente (12) die erste Komponente (11) umhüllt, und ein zweiter Umgehungskanal (15), der mit dem ersten Umgehungskanal (114) in Verbindung steht, zwischen dem Vorsprungsteil (112, 112') der ersten Komponente (11) und der zweiten Komponente (12) ausgebildet ist;
einen ersten Dichtungsring (13), der um eine Seitenfläche des oberen Teils (111) der ersten Komponente (11) angeordnet ist, am Verlängerungsteil (122) der zweiten Komponente (12) anliegt und einen ersten Spalt abdichtet, der zwischen dem oberen Teil (111) und dem Verlängerungsteil (122) definiert ist, und
ein elastisches Stück (14), das den Vorsprungsteil (112, 112') der ersten Komponente (11) umgibt und zwei Enden aufweist, und wobei die Enden des elastischen Stücks (14) mit dem oberen Teil (111) der ersten Komponente (11) bzw. dem unteren Teil (121) der zweiten Komponente (12) verbunden sind;
wobei das mehrstufige Luftdruckventil (1, 1') **dadurch gekennzeichnet ist, dass**:
eine breiteste radiale Breite des Vorsprungsteils (112, 112') der ersten Komponente (11) kleiner ist als eine radiale Breite des Auslasskanals der zweiten Komponente (12), derart dass der Vorsprungsteil (112, 112') im Auslasskanal vor- und rückwärts geschoben werden kann;
das mehrstufige Luftdruckventil (1, 1') ferner einen zweiten Dichtungsring (13) umfasst, wobei der zweite Dichtungsring (13) um eine Seitenfläche des Vorsprungsteils (112, 112') der ersten Komponente (11) angeordnet ist; und
der zweite Luftauslass (1141) sich zwischen dem ersten und dem zweiten Dichtungsring (13) befindet;
wobei
vor dem Zusammendrücken der ersten Komponente (11) der zweite Dichtungsring (13) außerhalb des Auslasskanals der zweiten Komponente (12) positioniert und der zweite Umgehungskanal (15) offen ist; und
bei Zusammendrücken der ersten Komponente (11) der Vorsprungsteil (112, 112') der ersten Komponente (11) zum Auslasskanal der zweiten Komponente (12) geschoben wird und der zweite Umgehungskanal (15) geschlossen wird, wenn der zweite Dichtungsring (13) in den Auslasskanal geschoben wird und an den unteren Teil (121) der zweiten Komponente (13) stößt, um einen zweiten Spalt abzudichten, der zwischen dem Vorsprungsteil (112, 112') und dem unteren Teil (121) definiert ist.

2. Mehrstufiges Luftdruckventil (1') nach Anspruch 1, wobei die erste Komponente (11) sich zwischen dem zweiten Luftauslass (1141) und dem ersten Luftauslass (1132) zu einer konisch zulaufenden Form ausgestaltet, um verschiedene Spalte zwischen der ersten Komponente (11) und der zweiten Komponente (12) zu bilden.

3. Verstellfreier mehrstufiger Prothesen-Luftzylinder, der auf ein Prothesengelenk anwendbar ist und umfasst:
ein mehrstufiges Luftdruckventil (1, 1') nach Anspruch 1;
einen Luftzylinderkörper (2), der einen unteren Luftweg (22) bildet, wobei eine Luftkammer (21) innerhalb des Luftzylinderkörpers (2) ausgebildet ist;
einen Kolben (3), wobei ein Ende des Kolbens (3) verschiebbar in der Luftkammer (21) angeordnet ist, und das andere Ende des Kolbens (3) sich aus dem Luftzylinderkörper (2) erstreckt, wobei der Kolben (3) die Luftkammer (21) in eine obere Luftkammer (211) und eine untere Luftkammer (212) darin teilt, wobei ein oberer Luftweg (31) innerhalb des Kolbens (3) ausgebildet ist, ein Ende des oberen Luftwegs (31) mit der Außenseite verbunden ist, und das andere Ende des oberen Luftwegs (31) mit der oberen Luftkammer (211) verbunden ist, wobei die untere Luftkammer (212) mit dem unteren Luftweg (22) in Verbindung steht, und wobei der Kolben (3) zum Durchführen einer Hin- und Herbewegung in der Luftkammer (21) ausgelegt ist;
ein erstes Sperrventil (4), wobei das erste Sperrventil (4) im Kolben (3) angeordnet ist, um die obere Luftkammer (211) optional mit der unteren Luftkammer (212) zu verbinden; und
wobei das mehrstufige Luftdruckventil (1, 1') im unteren Luftweg (22) angeordnet ist.

4. Verstellfreier mehrstufiger Prothesen-Luftzylinder nach Anspruch 3, ferner umfassend ein zweites Sperrventil (5), wobei das zweite Sperrventil (5) im oberen Luftweg (31) angeordnet ist, so dass die Luft nur von der Außenseite des Luftzylinderkörpers (2) in die obere Luftkammer (211) in einer Richtung strömen kann.

5. Verstellfreier mehrstufiger Prothesen-Luftzylinder nach Anspruch 3, wobei das mehrstufige Luftdruckventil (1, 1') mit mindestens einem zweiten Dichtungsring versehen ist, um den Spalt zwischen dem mehrstufigen Luftdruckventil und dem unteren Luftweg (22) zu schließen.

## Revendications

1. Soupape de pression d'air multi-niveaux (1, 1') disposée dans le passage d'air d'un vérin pneumatique de prothèse, la soupape de pression d'air multi-niveaux (1, 1') comprenant :
un premier composant (11) comprenant une partie supérieure (111) et une partie en saillie (112, 112'), la partie en saillie (112, 112') s'étendant axialement à partir de la partie supérieure (111), un intérieur du premier composant (11) comprenant un canal principal (113) et un premier canal de dérivation (114), le canal principal (113) pénétrant à travers le premier composant (11) de la partie supérieure (111) à la partie en saillie (112, 112'), formant une entrée d'air (1131) sur une surface supérieure de la partie supérieure (11) du premier composant (11) et formant une première sortie d'air (1132) sur une surface inférieure de la partie en saillie (112, 112') du premier composant (11), et le premier canal de dérivation (114) communiquant avec le canal principal (113) et formant une seconde sortie d'air (1141) sur une surface latérale du premier composant (11) ;
un second composant (12) comprenant une partie inférieure (121), une partie d'extension (122) et un canal de sortie, la partie en saillie (112, 112') du premier composant (11) s'étendant vers la partie inférieure (121), la partie d'extension (122) s'étendant axialement à partir de la partie inférieure (121) vers la partie supérieure (111) du premier composant (11), le canal de sortie pénétrant à travers la partie d'extension (122), le second composant (12) étant emmanché sur le premier composant (11), et un second canal de dérivation (15) communiquant avec le premier canal de dérivation (114) étant formé entre la partie en saillie (112, 112') du premier composant (11) et le second composant (12) ;
une première bague d'étanchéité (13) disposée autour d'une surface latérale de la partie supérieure (111) du premier composant (11), en butée contre la partie d'extension (122) du second composant (12), et assurant l'étanchéité d'un premier intervalle défini entre la partie supérieure (111) et la partie d'extension (122) ; et
une pièce élastique (14) entourant la partie en saillie (112, 112') du premier composant (11) et ayant deux extrémités, et les extrémités de la pièce élastique (14) étant reliées respectivement à la partie supérieure (111) du premier composant (11) et à la partie inférieure (121) du second composant (12) ;
la soupape de pression d'air multi-niveaux (1, 1') est **caractérisée par le fait que** :
une largeur radiale la plus large de la partie en saillie (112, 112') du premier composant (11) est plus petite qu'une largeur radiale du canal de sortie du second composant (12) de telle sorte que la partie en saillie (112, 112') est capable de coulisser en va-et-vient dans le canal de sortie ;
la soupape de pression d'air multi-niveaux (1, 1') comprend en outre une seconde bague d'étanchéité (13), la seconde bague d'étanchéité (13) étant disposée autour d'une surface latérale de la partie en saillie (112, 112') du premier composant (11) ; et
la seconde sortie d'air (1141) est située entre les première et seconde bagues d'étanchéité (13) ;
dans laquelle
avant que le premier composant (11) ne soit comprimé, la seconde bague d'étanchéité (13) est positionnée hors du canal de sortie du second composant (12) et le second canal de dérivation (15) est ouvert ; et
lorsque le premier composant (11) est comprimé, la partie en saillie (112, 112') du premier composant (11) coulisse vers le canal de sortie du second composant (12) et le second canal de dérivation (15) est fermé à mesure que la seconde bague d'étanchéité (13) coulisse dans le canal de sortie et vient en butée contre la partie inférieure (121) du second composant (13) pour assurer l'étanchéité d'un second intervalle défini entre la partie en saillie (112, 112') et la partie inférieure (121).

2. Soupape de pression d'air multi-niveaux (1') selon la revendication 1, dans laquelle le premier composant (11) suit une forme effilée entre la seconde sortie d'air (1141) et la première sortie d'air (1132) de façon à former des intervalles variables entre le premier composant (11) et le second composant (12).

3. Vérin pneumatique de prothèse multi-niveaux ne nécessitant pas de réglage, pouvant être appliqué à une articulation de prothèse, comprenant :
une soupape de pression d'air multi-niveaux (1, 1') selon la revendication 1 ;
un corps de vérin pneumatique (2) formant un passage d'air inférieur (22), une chambre à air (21) étant formée à l'intérieur du corps de vérin pneumatique (2) ;
un piston (3), une extrémité du piston (3) étant disposée de manière coulissante dans la chambre à air (21), et l'autre extrémité du piston (3) s'étendant hors du corps de vérin pneumatique (2), le piston (3) divisant la chambre à air (21) en une chambre à air supérieure (211) et une chambre à air inférieure (212), un passage d'air supérieur (31) étant formé à l'intérieur du piston (3), une extrémité du passage d'air supérieur (31) étant reliée à l'extérieur et l'autre extrémité du passage d'air supérieur (31) étant reliée à la chambre à air supérieure (211), la chambre à air inférieure (212) communiquant avec le passage d'air inférieur (22), et le piston (3) étant adapté pour réaliser un mouvement de va-et-vient dans la chambre à air (21) ;
un premier clapet anti-retour (4), le premier clapet anti-retour (4) étant disposé dans le piston (3) pour facultativement relier la chambre à air supérieure (211) à la chambre à air inférieure (212) ; et
la soupape de pression d'air multi-niveaux (1, 1') étant disposée dans le passage d'air inférieur (22).

4. Vérin pneumatique de prothèse multi-niveaux ne nécessitant pas de réglage selon la revendication 3, comprenant en outre un second clapet anti-retour (5), le second clapet anti-retour (5) étant disposé dans le passage d'air supérieur (31) de telle sorte que l'air peut s'écouler uniquement à partir de l'extérieur du corps de vérin pneumatique (2) jusqu'à la chambre à air supérieure (211) de manière unidirectionnelle.

5. Vérin pneumatique de prothèse multi-niveaux ne nécessitant pas de réglage selon la revendication 3, dans lequel la soupape de pression d'air multi-niveaux (1, 1') comporte au moins une seconde bague d'étanchéité pour fermer l'intervalle entre la soupape de pression d'air multi-niveaux et le passage d'air inférieur (22).
